# EUROPEAN PATENT SPECIFICATION

(11) **EP 4 452 346 B1**
(45) Date of publication and mention of the grant of the patent: **18.06.2025**
(21) Application number: 22838988.8
(22) Date of filing: 20.12.2022
(51) Int. Cl.: A61L 27/16, A61L 27/18, A61L 27/24, A61L 27/38

(54) **METHOD AND KIT FOR TREATING A DAMAGED CONNECTIVE TISSUE**
VERFAHREN UND KIT ZUR BEHANDLUNG EINES BESCHÄDIGTEN BINDEGEWEBES
PROCÉDÉ ET KIT DE TRAITEMENT D'UN TISSU CONJONCTIF ENDOMMAGÉ

(30) Priority: 22.12.2021 IL 28928121
(43) Date of publication of application: 30.10.2024
(73) Proprietor: Reddress Ltd., 3701142 Pardes Hana (IL)
(72) Inventor: KUSHNIR, Alon, 3780800 Givat Ada (IL)
(74) Representative: Maiwald GmbH
(86) International application number: PCT/IL2022/051350
(87) International publication number: WO 2023/119274

(56) References cited:
- EP-A1- 3 481 445
- WO-A2-2011/110948
- US-A1- 2021 315 587
- EVROVA OLIVERA ET AL: "Elastic and surgeon friendly electrospun tubes delivering PDGF-BB positively impact tendon rupture healing in a rabbit Achilles tendon model", BIOMATERIALS, ELSEVIER, AMSTERDAM, NL, vol. 232, 23 December 2019 (2019-12-23), XP086001944, ISSN: 0142-9612, [retrieved on 20191223], DOI: 10.1016/J.BIOMATERIALS.2019.119722

## Description

### TECHNOLOGICAL FIELD

The present disclosure is in the field of medical treatment of damaged connective tissues.

### BACKGROUND ART

References considered to be relevant as background to the presently disclosed subject matter are listed below:
- WO 2019/058373
- WO 2019/058375
- US 9,180,142

Acknowledgement of the above references herein is not to be inferred as meaning that these are in any way relevant to the patentability of the presently disclosed subject matter.

EVROVA OLIVERA ET AL: "Elastic and surgeon friendly electrospun tubes delivering PDGF-BB positively impact tendon rupture healing in a rabbit Achilles tendon model", BIOMATERIALS, ELSEVIER, AMSTERDAM, NL, vol. 232, 23 December 2019, concerns the treatment of tendon ruptures using hollow tubes placed over the damaged tissue and discloses tubes delivering PDGF-BB as implants for improving tendon rupture healing in a rabbit model.

### GENERAL DESCRIPTION

The present disclosure provides a combination and a kit for inducing growth or regeneration of a damaged connective tissue of a subject, or the treatment thereof by applying a clot of blood that is withdrawn from the subject. The connective tissue can be tendon, for example. By one implementation of the present invention, the blood that is withdrawn from the subject is introduced to a lumen of a connective tissue enveloping hollowed element that envelopes the damaged portion of the connective tissue while controllably coagulating within the lumen to form a clot on the damaged portion of the connective tissue. The clot, while in physical contact with the damaged portion of the connective tissue, enhances the rehabilitation of the connective tissue and may partially or fully restore its functionality.

Thus, an aspect of the present disclosure provides a combination for use in method for regenerating a connective tissue in or across a damaged connective tissue portion. The method includes fitting a connective tissue enveloping hollowed element having a lumen, such as a connective tissue guidance conduit or to envelope the connective tissue portion such that it resides within said lumen. The connective tissue enveloping hollowed element is designed to envelope the damaged connective tissue portion and to maintain a space between a peripheral wall thereof and the damaged connective tissue to allow introduction of coagulating blood withdrawn from the subject to the space to form a blood clot on the damaged connective tissue. Thus, the method further includes introducing a mixture of whole blood withdrawn from the subject with one or more coagulating agents and/or anti-anti coagulating agents into said lumen prior to its complete coagulation of the mixture.

It is to be noted, for any aspect of the present disclosure, that the whole blood may be withdrawn from the subject and initially mixed with anti-coagulating agents to prevent its natural coagulation. At a desired timing, the whole blood is mixed with one or more coagulating agents and/or anti-anti coagulating agents to controllably coagulate the whole blood on the damaged connective tissue portion to form the desired blood clot thereon.

In some embodiments of the method, said fitting comprises placing a film or sheet over said portion and enveloping said portion with said sheet to form said connective tissue enveloping hollowed element.

Also described is a method for inducing growth of connective tissue fibers across a damaged connective tissue portion. The method includes fitting a connective tissue enveloping hollowed element having a lumen, such as a connective tissue guidance conduit to envelope said connective tissue portion such that it resides within said lumen; and introducing a mixture of whole blood withdrawn from the subject with one or more coagulating agents and/or anti-anti coagulating agents into said lumen prior to its complete coagulation of the mixture.

In some embodiments of the method, the connective tissue enveloping hollowed element is a connective tissue guidance conduit.

In some embodiments of the method, the damaged connective tissue portion comprises cut connective tissue fibers.

In some embodiments of the method, the connective tissue portion includes two cut connective tissue stumps that are surgically joined or brought into proximity.

In some embodiments of the method, said fitting includes placing a film or sheet over said portion and enveloping said portion with said sheet to form said conduit.

In some embodiments of the method, said fitting comprises introducing the two connective tissue stumps into said connective tissue enveloping hollowed element, one from each end of the conduit; and bringing the two stumps into proximity to one another within said connective tissue enveloping hollowed element.

In some embodiments of the method, said bringing comprises securing the two stumps within said connective tissue enveloping hollowed element by suturing.

In some embodiments of the method, said introducing comprises filling a portion of the conduit's lumen with the mixture such that it comes into contacts with said connective tissue portion.

In some embodiments of the method, the connective tissue portion includes two cut connective tissue stumps and said introducing is carried out such that said mixture comes into tight contact with said stumps.

In some embodiments, the method further includes saturating the blood, prior to said introducing, with an oxygen-rich gas, e.g., oxygen-enriched air or pure oxygen.

In some embodiments, the method further includes mixing the blood, prior to said introducing, with one or more autologous cells. In some embodiments, said one or more autologous cells comprise Schwann cell.

In some embodiments of the method, the connective tissue is a tendon or a ligament. For example, the connective tissue can be the anterior cruciate ligament, or any tendon associated in the knee.

Also described is a method for treating damaged connective tissue of a subject or facilitating connective tissue regeneration, regrowth or rehabilitation process having a cut connective tissue with first and second connective tissue stumps. The method includes fitting and securing, e.g. by suturing, over said first connective tissue stump a first open end portion of a connective tissue guidance conduit, and fitting and securing over said second connective tissue stump a second open end portion of said connective tissue guidance conduit. The connective tissue guidance conduit defines a lumen extending between said first and second open end portions. The method further includes mixing whole blood withdrawn from the subject with one or more coagulating agents and/or anti-anti coagulating agents and introducing it into said lumen prior to its complete coagulation, namely while it is still in a flowable form, and permitting the blood to coagulate within said lumen.

In some embodiments of the method, said securing comprises suturing said first and second connective tissue stumps to the first and second open end portions, respectively.

In some embodiments of the method, said introducing comprises filling a portion of the lumen with blood such that it contacts both first and second connective tissue stumps, namely filling the gap or space between the first and second connective tissue stumps with blood and creating a continuous matrix of connective tissue stumps and blood.

In some embodiments, the method further includes saturating the blood, prior to said introducing, with oxygen.

In some embodiments, the method further includes, prior to said introducing, adding to or saturating the blood with keratin, stem cells (of all types), growth factors, Collagen, bone marrow or other gases or materials.

In some embodiments of the method, said introducing comprises inserting a blood applicator into the lumen and injecting the blood into the lumen by said blood applicator, e.g. a syringe.

In some embodiments, the method further includes mixing the blood, prior to said introducing, with one or more autologous cells. In some embodiments, said one or more autologous cells comprise Schwann cell.

In some embodiments of the method, the connective tissue guidance conduit is made of or of the type of collagen, polyglycolic acid (PGA), polycaprolactone (PCL), polyvinyl alcohol (PVA), and porcine small intestine submucosa (SIS).

In some embodiments of the method, the connective tissue is a tendon or a ligament.

Yet another aspect of the present disclosure provides a kit for treating, inducing growth, or regenerating a damaged connective tissue portion of a subject. The kit comprising a connective tissue enveloping hollowed element having a lumen for enveloping said damaged connective tissue portion such that said portion resides in at least a portion of said lumen; one or more blood withdrawal devices for allowing withdrawal of blood from the subject; one or more blood collection receptacles for receiving the blood withdrawn from the subject; a coagulation assembly configured for permitting mixture of the withdrawn blood with one or more coagulation agents for initiating coagulation process of the withdrawn blood; and an applicator for introducing the incomplete coagulated blood into said lumen.

In some embodiments of the kit, the one or more collection receptacles are vacuum tubes.

In some embodiments of the kit, the coagulation assembly comprises a volume for introducing the withdrawn blood, said volume is contactable with one or more coagulation agents or anti-anticoagulation agents.

In some embodiments, the kit further includes one or more anticoagulation agents for mixing with the withdrawn blood.

In some embodiments of the kit, the one or more anti-coagulation agents are comprised within said one or more blood collection receptacles.

In some embodiments of the kit, the anticoagulation agent is selected from a group consisting of: ACD-A (Anticoagulant Citrate Dextrose, Solution A), EDTA (ethylenediaminetetraacetic Acid), EGTA (ethylene glycol tetraacetic acid), a citrate, Heparin and oxalate.

In some embodiments of the kit, the one or more coagulation agents or anti-anticoagulating agents are selected from: Kaolin, Ca²⁺, e.g. in the form of calcium salts such as Calcium Gluconate, Mg²⁺, negatively charged phospholipid (PL) and protamine sulfate.

In some embodiments, the kit further includes a film or sheet for placing over said damaged connective tissue portion for forming said connective tissue enveloping hollowed element.

In some embodiments of the kit, the connective tissue enveloping hollowed element is a connective tissue guidance conduit.

In some embodiments of the kit, said damaged connective tissue portion is a cut connective tissue having a first and second connective tissue stumps.

In some embodiments, the kit further includes securing or retaining elements for securing said damaged connective tissue portion to the connective tissue enveloping hollowed element.

In some embodiments of the kit, the connective tissue is a tendon or a ligament.

In some embodiments, the kit is intended for use in a method for treating, inducing growth, or regenerating a damaged connective tissue portion. The method includes (i) enveloping said damaged connective tissue portion with a connective tissue enveloping hollowed element having a lumen such that said damaged connective tissue portion resides in said lumen; (ii) withdrawing whole blood from the subject; (iii) mixing the subject's blood with one or more coagulation agents or anti-anticoagulation agents; (iv) prior to complete coagulation of the blood, introducing the blood with the coagulation agent into said lumen; and (v) permitting the blood to coagulate in said lumen.

In some embodiments, the kit is intended for use in a method according to any one of the above-described embodiments of the method.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1D** are schematic illustrations exemplifying different stages of the method of inducing growth or regenerating a damaged or cut connective tissue according to aspects of the present disclosure.
**Figs. 2A-2C** are schematic illustrations exemplifying different stages of the method of inducing growth or regenerating a damaged connective tissue according to an aspect of the present disclosure.

### DETAILED DESCRIPTION

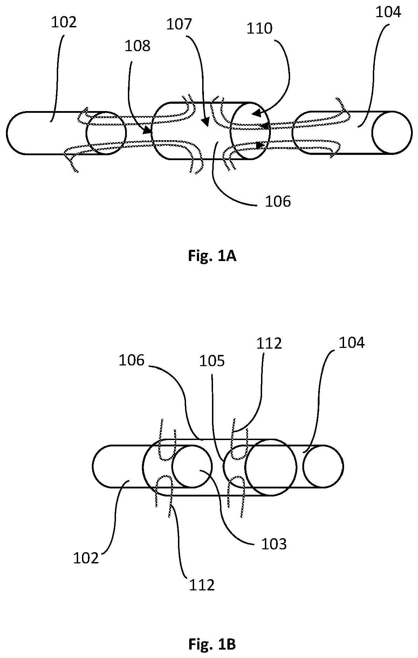

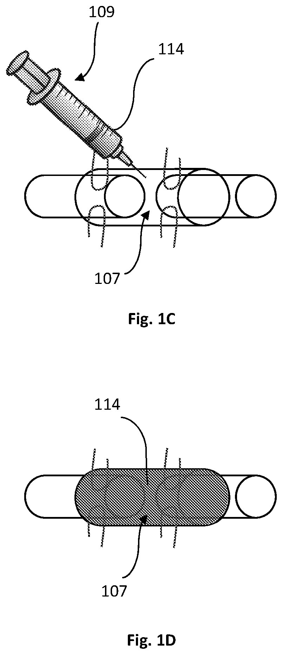

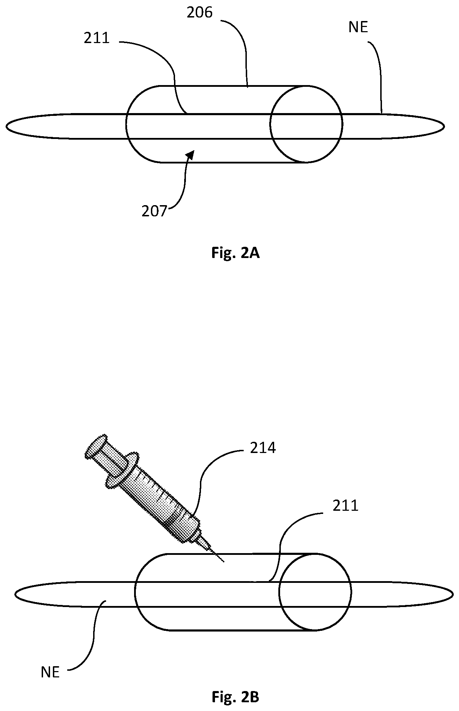

Reference is being made to **Figs. 1A-1D****,** which are schematic illustrations exemplifying different stages of the method of inducing growth or regenerating a damaged or cut connective tissue according to aspects of the present disclosure. **Fig. 1A** shows a first and second stumps **102** and **104** of a cut connective tissue of a subject being treated. A connective tissue guidance conduit **106** is a hollowed element having a lumen **107** extending between first and second open ends **108** and **110** and is configured to receive each of the stumps **102/104** through a respective open end such that the connective tissue guidance conduit **106** is fitted over the first and second stumps **102** and **104.** In **Figs. 1A-1D** and **Figs. 2A-2B****,** the connective tissue guidance conduit is presented as transparent for ease of description, however it is to be noted that the connective tissue guidance conduit can be made of materials that have any degree of transparency between full transparency and full opaque. In **Fig. 1A** the connective tissue stumps **102/104** are being introduced via the first and the second open ends **108** and **110,** respectively, and being secured, e.g. by suturing process such that portions of the connective tissue guidance conduit envelopes the stumps. **Fig. 1B** shows the stumps **102** and **104** within the connective tissue guidance conduit **106** and secured in position by securing elements **112** such that a portion that includes the end of each of the stumps **103** and **105** is within the lumen of the connective tissue guidance conduit **106** and each of the stumps extends beyond the respective opening. A mixture **114** of whole blood withdrawn from the subject with one or more coagulating agents and/or anti-anti coagulating agents is introduced into the lumen **107,** prior to the complete coagulation of the mixture, namely when the blood is still in a flowable form, as can be seen in **Fig. 1C****.** The introduction of the mixture into the lumen can be performed by a syringe **109** or any suitable means. The lumen **107** is filled with the mixture such that the mixture contacts at least portions of each of the stumps. **Fig. 1D** shows that the mixture forms a continuous matrix linking the two stumps **102/104.** The coagulation process of the mixture proceeds when it is within the lumen **107** until a clot is formed that contacts or envelopes one or two of the stumps **102/104.** To retain the mixture within the lumen **107,** the first and the second open ends **108** and **110** may be sealed, e.g. by a cloth, a bandage or any suitable sealing means.

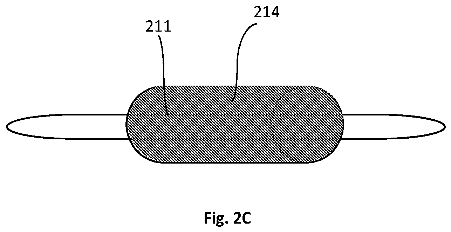

Reference is now being made to **Figs. 2A-2C****,** which are schematic illustrations of a non-limiting example of an embodiment according to an aspect of the present disclosure. **Fig. 2A** shows a connective tissue guidance conduit **206** that is fitted over a damaged portion **211** of a connective tissue **NE** of a subject. Once the connective tissue guidance conduit **206** is in place, a mixture **214** of whole blood withdrawn from the subject with one or more coagulating agents and/or anti-anti coagulating agents is introduced into a lumen **207** of the connective tissue guidance conduit **206,** prior to the complete coagulation of the mixture, namely when the blood is still in a flowable form, as can be seen in Fig. **2B****.** The mixture **214** fills the lumen **207** such that it contacts or envelopes the damaged portion **211** of the connective tissue **NE** and is maintained in the lumen to form a clot that contacts or envelopes the damaged portion **211** of the connective tissue **NE,** as can be seen in **Fig. 2C****.** In time, the clot induces regeneration or growth of the damaged connective tissue.

## Claims

1. A combination for use in regenerating a connective tissue in or across a damaged connective tissue portion, comprising:
a connective tissue guidance conduit configured for enveloping said connective tissue portion;
one or more coagulating agents and/or anti-anti coagulating agents; and
means for introducing a mixture of (i) whole blood withdrawn from the subject and (ii) one or more coagulating agents and/or anti-anti coagulating agents, into a lumen defined by said conduit when enveloping said connective tissue portion prior to the complete coagulation of the mixture.

2. The combination of claim 1, wherein said connective tissue guidance conduit is a film or sheet configured for placement over and enveloping said portion to form said conduit.

3. The combination of claim 1 or 2, for use in regenerating cut connective tissue fibers.

4. The combination of claim 3, for use in regenerating two cut connective tissue stumps that are surgically joined or brought into proximity.

5. The combination of any one of claims 1-4, comprising means for saturating the blood, prior to said introducing, with an oxygen-rich gas.

6. The combination of any one of claims 1-5, comprising a blood applicator configured for injecting the blood into said lumen.

7. The combination of any one of claims 1-6, for use in treating a damaged ligament or a damaged tendon.

8. A kit for use in inducing growth or regeneration of a damaged connective tissue portion of a subject, the kit comprising:
a connective tissue enveloping hollowed element for enveloping a damaged connective tissue portion;
one or more blood withdrawal devices for withdrawing blood from the subject;
one or more blood collection receptacles for receiving the blood withdrawn from the subject;
one or more coagulating agents and/or anti-anti coagulating agents;
a coagulation assembly configured for mixing the withdrawn blood with the one or more coagulation agents and/or anti-anti coagulating agents for initiating a coagulation process of the withdrawn blood; and
an applicator for introducing the incomplete coagulated blood into said lumen.

9. The kit of claim 8, for use in a method that comprises
fitting said element over said damaged connective tissue portion to envelope said tissue portion to thereby define a lumen, and
introducing a mixture of (i) whole blood withdrawn from the subject and (ii) one or more coagulating agents and/or anti-anti coagulating agents into said lumen prior to complete coagulation of the mixture.

10. The kit of claim 8 or 9, wherein said one or more collection receptacles are vacuum tubes.

11. The kit of any one of claims 8 to 10, wherein the coagulation assembly comprises a volume for introducing the withdrawn blood, said volume comprises one or more coagulation agents or anti-anticoagulation agents.

12. The kit of any one of claims 8 to 11, wherein said element is a film or sheet for placing over and enveloping said damaged connective tissue.

13. The kit of any one of claims 8 to 12, wherein said connective tissue enveloping hollowed element is a tissue guidance conduit.

14. The kit of any one of claims 8 to 13, comprising securing elements for securing said damaged connective tissue portion to the connective tissue enveloping hollowed element.

15. The kit of any one of claims 8 to 14, for use in a method for treating, inducing growth, or regenerating a damaged connective tissue portion, that comprises:
(i) enveloping said damaged connective tissue portion with a connective tissue enveloping hollowed element having a lumen such that said damaged connective tissue portion resides in said lumen;
(ii) withdrawing whole blood from the subject;
(iii) mixing the subject's blood with one or more coagulation agents or anti-anticoagulation agents;
(iv) prior to complete coagulation of the blood, introducing the blood with the coagulation agent into said lumen; and
(v) permitting the blood to coagulate in said lumen.

## Patentansprüche

1. Kombination zur Verwendung beim Regenerieren eines Bindegewebes in einem beschädigten Bindegewebsabschnitt oder über diesen hinweg, umfassend:
einen Bindegewebe-Führungskanal, der zum Umhüllen des Bindegewebsabschnitts ausgelegt ist;
ein oder mehrere Gerinnungsmittel und/oder anti-Gerinnungshemmer sowie
Mittel zum Einbringen einer Mischung aus (i) Vollblut, das dem Patienten abgenommen wurde, und (ii) einem oder mehreren Gerinnungsmitteln und/oder anti-Gerinnungshemmern in ein Lumen, das von dem Kanal definiert wird, wenn er den Bindegewebsabschnitt umhüllt, bevor die Mischung vollständig geronnen ist.

2. Kombination nach Anspruch 1, wobei der Bindegewebe-Führungskanal eine Folie oder Bahn ist, die zum Platzieren über dem Abschnitt und Umhüllen des Abschnitts ausgelegt ist, damit der Kanal ausgebildet wird.

3. Kombination nach Anspruch 1 oder 2 zur Verwendung beim Regenerieren von zertrennten Bindegewebsfasern.

4. Kombination nach Anspruch 3 zur Verwendung beim Regenerieren von zwei zertrennten Bindegewebsstümpfen, die operativ verbunden oder nahe zueinander gebracht werden.

5. Kombination nach einem der Ansprüche 1-4, umfassend Mittel zum Sättigen des Bluts mit einem sauerstoffreichen Gas vor dem Einbringen.

6. Kombination nach einem der Ansprüche 1-5, umfassend eine Blutverabreichungsvorrichtung, die zum Einspritzen des Bluts in das Lumen ausgelegt ist.

7. Kombination nach einem der Ansprüche 1-6 zur Verwendung bei der Behandlung eines beschädigten Bands oder einer beschädigten Sehne.

8. Kit zur Verwendung bei der Förderung des Wachstums oder der Regeneration eines beschädigten Bindegewebsabschnitts eines Patienten, wobei das Kit Folgendes umfasst:
ein hohles Bindegewebe-Umhüllelement zum Umhüllen eines beschädigten Bindegewebsabschnitts;
ein oder mehrere Blutabnahmevorrichtungen zum Abnehmen von Blut aus dem Patienten;
ein oder mehrere Blutentnahmebehältnisse zum Aufnehmen des Bluts, das dem Patienten abgenommen wurde;
ein oder mehrere Gerinnungsmittel und/oder anti-Gerinnungshemmer;
eine Gerinnungsanordnung, die zum Mischen des abgenommenen Bluts mit dem einen oder den mehreren Gerinnungsmitteln und/oder anti-Gerinnungshemmern zum Auslösen eines Gerinnungsvorgangs in dem abgenommenen Blut ausgelegt ist; und
eine Verabreichungsvorrichtung zum Einbringen des unvollständig geronnenen Bluts in das Lumen.

9. Kit nach Anspruch 8 zur Verwendung in einem Verfahren, das Folgendes umfasst:
Anlegen des Elements über dem beschädigten Bindegewebsabschnitt zum Umhüllen des Gewebeabschnitts und
dadurch Definieren eines Lumens und
Einbringen einer Mischung aus (i) Vollblut, das dem Patienten abgenommen wurde, und (ii) einem oder mehreren Gerinnungsmitteln und/oder anti-Gerinnungshemmern in das Lumen vor der vollständigen Gerinnung der Mischung.

10. Kit nach Anspruch 8 oder 9, wobei das eine oder die mehreren Entnahmebehältnisse Vakuumröhrchen sind.

11. Kit nach einem der Ansprüche 8 bis 10, wobei die Gerinnungsanordnung ein Volumen zum Einbringen des abgenommenen Bluts umfasst, das Volumen ein oder mehrere Gerinnungsmittel oder anti-Gerinnungshemmer umfasst.

12. Kit nach einem der Ansprüche 8 bis 11, wobei das Element eine Folie oder Bahn zum Platzieren über dem beschädigten Bindegewebe und Umhüllen des beschädigten Bindegewebes ist.

13. Kit nach einem der Ansprüche 8 bis 12, wobei das hohle Bindegewebe-Umhüllelement ein Gewebeführungskanal ist.

14. Kit nach einem der Ansprüche 8 bis 13, umfassend Sicherungselemente zum Sichern des beschädigten Bindegewebsabschnitt am hohlen Bindegewebe-Umhüllelement.

15. Kit nach einem der Ansprüche 8 bis 14 zur Verwendung in einem Verfahren zum Behandeln, Auslösen des Wachstums oder Regenerieren eines beschädigten Bindegewebsabschnitts, das Folgendes umfasst:
(i) Umhüllen des beschädigten Bindegewebsabschnitts mit einem hohlen Bindegewebe-Umhüllelement, das ein Lumen aufweist, so dass der beschädigte Bindegewebsabschnitt in dem Lumen liegt;
(ii) Abnehmen von Blut bei dem Patienten;
(iii) Vermischen des Bluts des Patienten mit einem oder mehreren Gerinnungsmitteln oder Anti-Gerinnungshemmern;
(iv) vor der vollständigen Gerinnung des Bluts, Einbringen des Bluts mit dem Gerinnungsmittel in das Lumen und
(v) Gerinnenlassen des Bluts in dem Lumen.

## Revendications

1. Combinaison destinée à être utilisée dans la régénération d'un tissu conjonctif dans ou à travers une portion de tissu conjonctif endommagé, comprenant :
un conduit de guide de tissu conjonctif configuré pour envelopper ladite portion de tissu conjonctif ;
un ou plusieurs agents coagulants et/ou agents anti-coagulants ; et
des moyens pour introduire un mélange de (i) sang entier retiré du sujet et (ii) un ou plusieurs agents coagulants et/ou agents anti-coagulants, dans une lumière définie par ledit conduit lorsqu'il enveloppe ladite portion de tissu conjonctif avant la coagulation complète du mélange.

2. Combinaison selon la revendication 1, dans laquelle ledit conduit de guide de tissu conjonctif est un film ou une feuille configuré pour le placement sur et l'enveloppement de ladite portion pour former ledit conduit.

3. Combinaison selon la revendication 1 ou 2, destinée à être utilisée dans la régénération de fibres de tissu conjonctif coupées.

4. Combinaison selon la revendication 3, destinée à être utilisée dans la régénération de deux moignons de tissu conjonctif coupés qui sont joints chirurgicalement ou portés à proximité.

5. Combinaison selon l'une quelconque des revendications 1 à 4, comprenant des moyens pour saturer le sang, avant ladite introduction, avec un gaz riche en oxygène.

6. Combinaison selon l'une quelconque des revendications 1 à 5, comprenant un applicateur de sang configuré pour injecter le sang dans ladite lumière.

7. Combinaison selon l'une quelconque des revendications 1 à 6, destiné à être utilisé dans le traitement d'un ligament endommagé ou d'un tendon endommagé.

8. Kit destiné à être utilisé dans l'induction de la croissance ou la régénération d'une portion de tissu conjonctif endommagé d'un sujet, le kit comprenant :
un élément vidé enveloppant un tissu conjonctif pour envelopper une portion de tissu conjonctif endommagé ;
un ou plusieurs dispositifs de retrait de sang pour retirer du sang du sujet ;
un ou plusieurs réceptacles de collecte de sang pour recevoir le sang retiré du sujet ;
un ou plusieurs agents coagulants et/ou anti-coagulants ;
un ensemble de coagulation configuré pour mélanger la sang retiré avec les un ou plusieurs agents coagulants et/ou agents anti-coagulants pour initier un processus de coagulation du sang retiré ; et
un applicateur pour introduire le sang coagullé incomplet dans ladite lumière.

9. Kit selon la revendication 8, destiné à être utilisé dans un procédé qui comprend
l'ajustement dudit élément sur ladite portion de tissu conjonctif endommagé pour envelopper ladite portion de tissu pour ainsi définir une lumière et
introduire un mélange de (i) sang entier du sujet et (ii) un ou plusieurs agents coagulants et/ou agents anti-coagulants dans ladite lumière avant la coagulation complète du mélange.

10. Kit selon la revendication 8 ou 9, dans lequel lesdits un ou plusieurs réceptacles de collecte sont des tubes à vide.

11. Kit selon l'une quelconque des revendications 8 à 10, dans lequel l'ensemble de coagulation comprend un volume pour introduire le sang retiré, ledit volume comprend un ou plusieurs agents de coagulation et/ou agents d'anti-coagulation.

12. Kit selon l'une quelconque des revendications 8 à 11, dans lequel ledit élément est un film ou une feuille pour placer sur et envelopper ledit tissu conjonctif endommagé.

13. Kit selon l'une quelconque des revendications 8 à 12, dans lequel ledit élément vidé enveloppant le tissu conjonctif est un conduit de guide de tissu.

14. Kit selon l'une quelconque des revendications 8 à 13, comprenant des éléments de fixation pour fixer ladite portion de tissu conjonctif endommagé à l'élément vidé enveloppant le tissu conjonctif.

15. Kit selon l'une quelconque des revendications 8 à 14, destiné à être utilisé dans un procédé pour le traitement, l'induction de croissance ou la régénération d'une portion de tissu conjonctif endommagé, qui comprend :
(i) envelopper ladite portion de tissu conjonctif endommagé avec un élément vidé enveloppant le tissu conjonctif ayant une lumière de telle manière que ladite portion de tissu conjonctif endommagé se situe dans ladite lumière ;
(ii) retirer du sang entier du sujet ;
(iii) mélanger le sang du sujet avec un ou plusieurs agents de coagulation ou agents d'anti-coagulation ;
(iv) avant la coagulation complète du sang, introduire le sang avec l'agent de coagulation dans ladite lumière ; et
(v) permettre au sang de coaguler dans ladite lumière.
